(19) Europäisches Patentamt
European Patent Office
Office européen des brevets

(11) EP 0 624 594 B1

(12) EUROPÄISCHE PATENTSCHRIFT

(45) Veröffentlichungstag und Bekanntmachung des Hinweises auf die Patenterteilung:
**11.08.1999 Patentblatt 1999/32**

(21) Anmeldenummer: **94106845.4**

(22) Anmeldetag: **02.05.1994**

(51) Int Cl.$^6$: **C07J 9/00**, A61K 31/575, C07J 41/00
// C07J31/00, C07J51/00, C07J43/00

(54) **Monomere Gallensäurederivate, Verfahren zu ihrer Herstellung und Verwendung dieser Verbindungen als Arzneimittel**

Monomeric bile acid derivatives, a process for their production, and their use as medicines

Dérivés des acides biliaires monomères, un procédé pour leur production et leur utilisation comme médicaments

(84) Benannte Vertragsstaaten:
**AT BE CH DE DK ES FR GB GR IE IT LI LU NL PT SE**

(30) Priorität: **08.05.1993 DE 4315439**

(43) Veröffentlichungstag der Anmeldung:
**17.11.1994 Patentblatt 1994/46**

(73) Patentinhaber: **HOECHST AKTIENGESELLSCHAFT
65929 Frankfurt am Main (DE)**

(72) Erfinder:
- **Glombik, Heiner, Dr.
D-65719 Hofheim am Taunus (DE)**
- **Enhsen, Alfons, Dr.
D-64572 Büttelborn (DE)**
- **Kramer, Werner, Dr. Dr.
D-55130 Mainz (DE)**
- **Wess, Günther, Dr.
D-63526 Erlensee (DE)**

(56) Entgegenhaltungen:

| | |
|---|---|
| **EP-A- 0 417 725** | **EP-A- 0 489 423** |
| **EP-A- 0 548 793** | **DE-B- 1 119 263** |
| **FR-A- 2 444 686** | **US-A- 3 859 437** |
| **US-A- 4 848 349** | |

- **TETRAHEDRON LETTERS, Bd. 33, Nr. 2, 1992 OXFORD GB, Seiten 195-198, G. WESS ET AL 'Modified bile acids: preparation of 7.alpha.,12.alpha.-dihydroxy-3.beta.- and 7.alpha.,12.alpha.-dihydroxy-3.alpha.-(2-hydroxyethoxy)-5.beta.-cholanic acid and their biological activity'**
- **JOURNAL OF MEDICINAL CHEMISTRY, Bd. 27, Nr. 6, Juni 1984 WASHINGTON US, Seiten 746-749, R. PELLICCIARI ET AL 'Bile Acids with a Cyclopropyl-Containing Side Chain. 1. Preparation and Properties of 3.alpha.,7.beta.-Dihydroxy-22,23-methylene-5.beta.-cholan-24-oic Acid'**
- **CHEMICAL ABSTRACTS, vol. 100, no. 1, 2.Januar 1984 Columbus, Ohio, US; abstract no. 003969, T C BARTHOLOMEW ET AL 'The effect of 3-sulfation and taurine conjugation on the uptake of chenodeoxycholic acid by rat hepatocytes' Seite 344; Spalte 1;**
- **CHEMICAL ABSTRACTS, vol. 95, no. 3, 20.Juli 1981 Columbus, Ohio, US; abstract no. 20651, G. A. DE WEERT ET AL 'Quantitative determination of fecal bile acids as their methyl ether methyl esters by the repetitive scan technique' Seite 306; Spalte 1;**
- **CHEMICAL ABSTRACTS, vol. 103, no. 1, 8.Juli 1985 Columbus, Ohio, US; abstract no. 004386, AMMON H V ET AL 'Effects of sulfodeoxycholate on rat and rabbit small intestine' Seite 404; Spalte 1;**
- **STEROIDS, Bd. 42, Nr. 5, November 1983 SAN FRANCISCO US, Seiten 575-592, N. MURATA ET AL 'DETERMINATION OF SULFATED AND NONSULFATED BILE ACIDS IN SERUM BY MASS FRAGMENTOGRAPHY'**
- **CHEMICAL ABSTRACTS, vol. 092, no. 9, 3.März 1980 Columbus, Ohio, US; abstract no. 073345, DE WITT E H ET AL 'Effects of sulfation patterns on intestinal transport of bile salt sulfate esters' Seite 404; Spalte 2;**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 624 594 B1

Printed by Jouve, 75001 PARIS (FR)

**(Forts. nächste Seite)**

- SYNTHETIC COMMUNICATIONS, Bd. 17, Nr. 9, 1987 Seiten 1111-1117, T. BANDIERA ET AL 'A CONVENIENT PROCEDURE FOR THE SYNTHESIS OF URSODEOXYCHOLIC ACID SULFATED DERIVATIVES'
- JOURNAL OF THE AMERICAN CHEMICAL SOCIETY, Bd. 74, Nr. 21, 5.November 1952 DC US, Seiten 5472-5474, J. C. BABCOCK ET AL 'Reductive Methylation of Steroid Ketones'
- STEROIDS: STRUCTURE, FUNCTION, AND REGULATION, Bd. 58, Nr. 2, Februar 1993 MA US, Seiten 79-86, I. MAZUMDER ET AL 'Bile acid transformations by Alcaligenes recti'
- TETRAHEDRON LETTERS, Bd. 34, Nr. 5, 29.Januar 1993 OXFORD GB, Seiten 817-818, G. WESS ET AL 'Preparation of 3.alpha.- and 3.beta.-(.omega.-aminoalkoxy)-7.alpha.,12. alpha.-dihydroxy-5.beta.-cholanoic acid esters: versatile shuttles for drug targeting'
- TETRAHEDRON LETTERS, Bd. 33, Nr. 30, 1992 OXFORD GB, Seiten 4329-4332, S. YAMADA ET AL 'Mild oxidation of aldehydes to the corresponding carboxylic acids and esters: alkaline iodine oxidation revisited'
- TETRAHEDRON, (INCL TETRAHEDRON REPORTS), Bd. 48, Nr. 10, 1992 OXFORD GB, Seiten 1837-1852, W. ZHOU ET AL 'Studies of steroidal plant-growth regulator 25. Concise stereoselective construction of side chain of brassinosteroid from the intact side chain of hyodeoxycholic acid: formal synthesis of brassinolide, 25-methylbrassinolide, 26,27-dimethylbrassinolide and their related compounds'
- CHEMICAL ABSTRACTS, vol. 55, no. 4, 20.Februar 1961 Columbus, Ohio, US; abstract no. 3648c, S. YASUO ET AL 'Basic bile acids. I. Synthesis and configuration of 3.alpha.aminocholanic acid' Spalte 3648;
- BULLETIN DE LA SOCIETE CHIMIQUE DE FRANCE (MEMOIRES), 1949 PARIS FR, Seiten 877-883, J. REDEL ET AL 'Amines cycliques des acides biliaires. Première partie : Monoamines'
- R. A. HILL ET AL 'DICTIONARY OF STEROIDS, INDEXES' 1991 , CHAPMAN & HALL , LONDON, GB * Seite 386, Spalte 2 - Seite 387, Spalte 2 *
- JOURNAL OF BIOLOGICAL CHEMISTRY, Bd. 267, Nr. 26, 1992 MD US, Seiten 18598-18604, W. KRAMER ET AL 'Liver specific drug targeting by coupling to bile acids'
- TETRAHEDRON LETTERS, Bd. 34, Nr. 5, Januar 1993 OXFORD GB, Seiten 819-822, WESS G ET AL 'Synthesis of bile acid - drug conjugates: potential drug shuttles for liver specific targeting'
- CHROMATOGRAPHIA , Bd. 32, Nr. 5/6, September 1991 Seiten 265-268, MUELLNER S ET AL 'Synthesis of affinity chromatography and electrophoresis matrixes for the purification of bile acid transport proteins'
- ARTERY (LEONIDAS, MICH.) , Bd. 3, Nr. 6, 1977 Seiten 553-575, QUACKENBUSH F W ET AL 'Arylsulfonate esters of fatty alcohols. II. Structural relation to hypocholesterolemic activity'
- CHEMICAL ABSTRACTS, vol. 110, no. 4, 23.Januar 1989 Columbus, Ohio, US; abstract no. 28956, N. HO ET AL 'Utilising bile acid carrier mechanisms to enhance liver and small intestine absoprtion' Seite 330; Spalte 2;
- HEPATOLOGY (BALTIMORE) , Bd. 6, Nr. 3, 1986 Seiten 444-449, SHERMAN I A ET AL 'Hepatic transport of fluorescent molecules: in vivo studies using intravital TV microscopy'
- ARZNEIMITTEL FORSCHUNG DRUG RESEARCH., Bd. 31, Nr. 10a, 1981 AULENDORF DE, Seiten 1863-1866, B. PALMIERI ET AL 'Clinical Research into the Hypolipemic and Platelet Antiaggregant Activity of Plafibride, Carried out in Double.blind Conditions and in Comparison with Clofibrate'
- JOURNAL OF MEDICINAL CHEMISTRY, Bd. 31, Nr. 6, 1988 WASHINGTON US, Seiten 1205-1209, SHIN-ICHI MORISHITA ET AL 'Synthesis and Hypolipidemic Activity of 2-Substituted Isobutyric Acid Derivatives'
- JOURNAL OF PHARMACEUTICAL SCIENCES, Bd. 73, Nr. 10, Oktober 1984 WASHINGTON US, Seiten 1482-1484, J. M. CHAPMAN ET AL 'Hypolipidemic Activity of Phthalimide Derivatives V: Reduced and Hydrolytic Products of Simple Cyclic Imides'
- CHEMICAL ABSTRACTS, vol. 073, no. 3, 1970 Columbus, Ohio, US; abstract no. 013070, KOCIAN J ET AL 'Effect of oral calcium administration on the cholesterol and phospholipid serum levels' Seite 220; Spalte 1;
- AM. J. PHYSIOL. GASTROINTESTINAL AND LIVER PHYSIOLOGY , Bd. 9, Nr. 5, Mai 1984 Seiten G477-G483, HARDISON W G M ET AL 'Specificity of an sodium-dependent taurocholate transport site in isolated rat hepatocytes'
- ARZNEIMITTEL FORSCHUNG DRUG RESEARCH., Bd. 35, Nr. 4, 1985 AULENDORF DE, Seiten 707-712, K. GREISEN ET AL 'Inhibition of 3H-Glibenclamide Binding to Sulfonylurea Receptors by Oral Antidiabetics'
- CHEMICAL ABSTRACTS, vol. 112, no. 21, 21.Mai 1990 Columbus, Ohio, US; abstract no. 193405, A. VILLEGAS-NAVARRO '9-Anthracenecarboxylic acid and hematological and biochemical variables in the rat' Seite 230; Spalte 1;
- BIOCHIMICA ET BIOPHYSICA ACTA, Bd. 1085, Nr. 2, 1991 Seiten 223-234, J. M. CRAWFORD ET AL 'Physical and biological properties of fluorescent dansylated bile salt derivatives: the role of steroid ring hydroxylation'




(Forts. nächste Seite)

- JOURNAL OF MEDICINAL CHEMISTRY, Bd. 37, Nr. 7, 1.April 1994 WASHINGTON US, Seiten 873-875, WESS G ET AL 'Specific Inhibitors of Ileal Bile Acid Transport'
- JOURNAL OF DRUG TARGETING, Bd. 1, Nr. 4, Dezember 1993 Seiten 347-359, DONG-CHOOL KIM ET AL 'Evaluation of the Bile Acid Transporter in Enhancing Intestinal Permeability to Renin-Inhibitory Peptides'

## Beschreibung

Gallensäuren werden in der Leber in mehreren enzymatischen Schritten aus Cholesterin synthetisiert. Sie werden in der Gallenblase gespeichert, aus der sie mit der Gallenflüssigkeit in den Dünndarm sezerniert werden. Dort erfüllen sie während des Verdauungsvorgangs wichtige physiologische Funktionen, z. B. als Cofaktoren oder pankreatischen Lipasen und als natürliche Detergentien bei der Resorption von Fetten und fettlöslichen Vitaminen. Durch aktive und passive Transportprozesse gelangt der größte Teil der Gallensäuren aus dem Dünndarm über das Pfortaderblut zurück in die Leber.

Gallensäurebindende Polymere werden seit längerer Zeit als Therapeutika eingesetzt. Sie finden Anwendung bei Erkrankungen, bei denen eine Hemmung der Gallensäurerückresorption wünschenswert ist. So kann bei erhöhtem Cholesterin-Blutspiegel durch Reduktion der im enterohepatischen Kreislauf befindlichen Gallensäuren eine erhöhte Synthese von Gallensäuren aus Cholesterin in der Leber induziert werden. Dies führt zu einer erhöhten LDL-Cholesterinaufnahme aus dem Blut in die Leber und zu einem beschleunigten LDL-Katabolismus. Der erzielte Effekt ist eine Senkung des atherogenen LDL-Cholesterins im Blut.

Die zu diesem Zweck als Arzneimittel verwendeten Polymere, z. B. Cholestyramin oder Colestipol, müssen in sehr hohe Tagesdosen, von 12 bis 30 g, verabreicht werden. Neben der hohen Dosierung erschweren Geschmack und Geruch die Akzeptanz bei Patient und Arzt.

Die genannte Polymere zeigen Nebenwirkungen aufgrund zu geringer Selektivität und zu hoher Bindung von Vitaminen und aufgrund von Wechselwirkungen mit simultan verabreichten Pharmaka. Weiterhin können sie die Gallensäurezusammensetzung in der Galle verändern. Diese Eigenschaften äußern sich in verschiedenen gastrointestinalen Störungen (z. B. Obstipation, Steatorrhoe), Avitaminosen und erhöhtem Cholelithiasis-Risiko.

In Tetrahedron Letters Bd. 34, Nr. 5, Januar 1993 GB, Seiten 819-822 sind 3-modifizierte Gallensäurederivate beschrieben, die Affinität zum Gallensäuretransportsystem aufweisen.

Überraschenderweise wurden nun neue monomere Gallensäurederivate gefunde, die den enterohepatischen Kreislauf der Gallensäuren unterbrechen können und die genannten Nachteile nicht besitzen.

Die Erfindung betrifft daher monomere Gallensäurederivate der Formel I

$$Z\text{-}X\text{-}GS \qquad I,$$

in der

GS einen Gallensäurerest mit acider Funktion in der Seitenkette oder dessen Salz bedeutet,

X ein kovalente Bindung oder eine Brückgruppe der Formel $(CH_2)_n$ mit n = 1 bis 10, wobei die Alkylenkette 1 bis 3 Sauerstoffatome, NH- oder

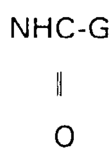-Gruppen

aufweisen kann, darstellt, wobei GS beliebig über X verbunden ist, und

Z

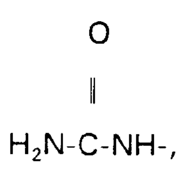, 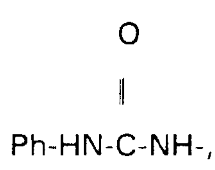, oder 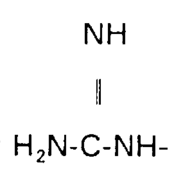

bedeutet.

Bevorzugt sind Verbindungen der Formel I, bei denen GS in 3-Position mit X verknüpft ist, wobei die Anknüpfung in α- oder β-Stellung erfolgt.

Unter acider Funktion wird insbesondere die COOH-Gruppe oder die Sulfonsäuregruppe verstanden.

Alkylreste sind geradkettig oder verzweigt.

Die erfindungsgemäßen Verbindungen der Formel (I) besitzen eine hohe Affinität zum spezifischen Gallensäuretransportsystem des Dünndarms und hemmen die Gallensäurerückresorption konzentrationsabhängig und kompetitiv.

Durch kompetitive Hemmung kann wesentlich selektiver in den enterohepatischen Kreislauf eingegriffen werden. Avitaminosen sind nicht zu erwarten, ebensowenig kommt es zu einer qualitativen Veränderung der Gallensäurezusammensetzung in der Galle. Mit den erfindungsgemäßen Verbindungen läßt sich eine gezielte Senkung des Serumcholesterinspiegels erreichen, ohne daß die bekannten Nebenwirkungen beobachtet werden. Wegen ihrer hohen Affinität zum Gallensäuretransportsystem kommt man zu sehr viel geringeren Tagesdosen als bei den im Handel befindlichen Polymeren; dies führt auch zu einer hohen Akzeptanz bei Patient und Arzt.

Die Verbindungen besitzen wertvolle pharmakologische Eigenschaften und eignen sich daher insbesondere als Hypolipidämika.

Die Erfindung betrifft daher auch Arzneimittel auf Basis der Verbindungen der Formel (I) sowie die Verwendung der Verbindungen als Arzneimittel, insbesondere zur Senkung des Cholesterinspiegels.

Die biologische Prüfung der erfindungsgemäßen Verbindungen erfolgte durch Ermittlung der Hemmung der [$^3$H]-Taurocholataufnahme in Bürstensaummembranvesikel des Ileums von Kaninchen. Der Hemmtest wurde wie folgt durchgeführt:

1. Präparation von Bürstensaummembranvesikeln aus dem Ileum von Kaninchen

Die Präparation von Bürstensaummembranvesikeln aus den Darmzellen des Dünndarm erfolgte mit der sogenannten $Mg^{2+}$-Präzipitationsmethode. Männliche Neuseeland-Kaninchen (2 bis 2,5 kg Körpergewicht) wurden durch intravenöse Injektion von 0,5 ml einer wäßrigen Lösung von 2,5 mg Tetracain HCl, 100 T 61$^R$ und 25 mg Mebezoniumjodid getötet. Der Dünndarm wurde entnommen und mit eiskalter physiologischer Kochsalzlösung gespült. Die terminalen 7/10 des Dünndarms (gemessen in oral rectaler Richtung, d. h. das terminale Ileum, welches das aktive $Na^+$-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/300 mM Mannit, 5 mM EGTA/10 mg/l Phenylmethylsulfonylfluorid/1 mg/l Trypsin Inhibitor v. Sojabohnen (32 U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, BRD) 3 Minuten bei 75 % max. Leistung unter Eiskühlung homogenisiert. Nach Zugabe von 3 ml 1 M $MgCl_2$-Lösung (Endkonzentration 10 mM) ließ man exakt 1 Minute bei 0°C stehen. Durch Zugabe von $Mg^{2+}$ aggregieren die Zellmembranen und präzipitieren mit Ausnahme der Bürstensaummembranen. Nach einer 15-minütigen Zentrifugation bei 3000 x g (5000 rpm, SS-34-Rotor) wurde der Niederschlag verworfen und der Überstand, der die Bürstensaummembranen enthielt, 30 Minuten bei 267000 x g (15000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M $MgCl_2$-Lösung und 15-minütiger Inkubationszeit bei 0°C wurde erneut 15 Minuten bei 3000 x g zentrifugiert. Der Überstand wurde anschließend nochmals 30 Minuten bei 46000 x g (15000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196°C in 4 mg Portionen in flüssigem Stickstoff aufbewahrt.

2. Hemmung der $Na^+$-abhängigen [$^3$H]Taurocholat-Aufnahme in Bürstensaummembranvesikel des Ileums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [$^3$H(G)]-Taurocholat (spezifische Aktivität: 2,1 Ci/mMol) /0,5 µl 10 mM Taurocholat/8,75 µl Natrium-Transport-Puffer (10 mM Tris/Hepes (pH 7,4)/100 mM Mannit/100 mM NaCl) (Na-T-P) bzw. 8,75 µl Kalium-Transport-Puffer (10 mM Tris/Hepes (pH 7,4)/ 100 mM Mannit/100 mM KCl) (K-T-P) und 80 µl der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm Ø, Millipore, Eschborn, BRD) filtriert. Durch Vermischung der Vesikel mit dem Inkubationsmedium wurde die Transportmessung gestartet. Die Konzentration an Taurocholat im Inkubationsansatz betrug 50 µM. Nach

der gewünschten Inkubationszeit (üblicherweise 1 Minute) wurde der Transport durch Zugabe von 1 ml eiskalter Stoplösung (10 mM Tris/Hepes (pH 7,4)/150 mM KCl) gestoppt. Die entstehende Mischung wurde sofort bei einem Vakuum von 25 bis 35 mbar über ein Membranfilter aus Cellulosenitrat (ME 25, 0,45 μm, 25 mm Durchmesser, Schleicher & Schuell, Dassell, BRD) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, BRD) aufgelöst und die Radioaktivität durch Flüssigkeits-Szintillationsmessung in einem Meßgerät TriCarb 2500 (Canberra Packard GmbH, Frankfurt, BRD) gemessen. Die gemessenen Werte wurden nach Eichung des Gerätes mit Hilfe von Standardproben und nach Korrektur evtl. vorhandener Chemilumineszenz als dpm (Decompositions per Minute) erhalten.

Die Kontrollwerte wurden jeweils in Na-T-P und K-T-P ermittelt. Die Differenz zwischen der Aufnahme in Na-T-P und K-T-P ergab den $Na^+$-abhängigen Transportanteil. Als $IC_{50}$ $Na^+$ wurde diejenige Konzentration an Inhibitor bezeichnet, bei der der $Na^+$-abhängige Transportanteil um 50 % - bezogen auf die Kontrolle - gehemmt war.

Die Tabelle zeigt die Meßwerte der Hemmung der [$^3$H]-Taurocholataufnahme in Bürstensaummembranvesikel des Ileums von Kaninchen. Angegeben sind die Quotienten aus den $IC_{50}$- bzw. $IC_{50Na}$-Werten des in jeder Vesikelpräparation als Standard untersuchten Taurochenodesoxycholats (TCDC) und der jeweiligen Substanz.

| Substanz aus Beispiel: | $\frac{IC_{50}\text{ (TCDC)}}{IC_{50}\text{ (Substanz)}}$ | $\frac{IC_{50Na}\text{ (TCDC)}}{IC_{50Na}\text{ (Substanz)}}$ |
|---|---|---|
| 3 | 0,4 | 0,35 |
| 4 | 0,77 | 0,69 |
| 18 | 0,47 | 0,42 |
| 21 | 0,34 | 0,33 |
| 33 | 0,33 | 0,35 |
| 35 | 1,0 | 1,02 |
| 36 | 0,19 | 0,20 |
| 38 | 0,49 | 0,41 |
| 40 | 0,52 | 0,50 |
| 43 | 0,78 | 0,73 |

Die Erfindung betrifft weiterhin die Verwendung der erfindungsgemäßen Verbindungen zur Herstellung eines Heilmittels.

Hierzu werden die Verbindungen der Formel I gelöst oder suspendiert in pharmakologisch unbedenklichen organischen Lösungsmitteln, wie ein- oder mehrwertigen Alkoholen, wie z. B. Ethanol oder Glycerin, in Triacetin, Ölen, z. B. Sonnenblumenöl, Lebertran, Ethern, wie z. B. Diethylenglykoldimethylether, oder auch Polyethern, z. B. Polyethylenglykol, oder auch in Gegenwart anderer z. B. Sonnenblumenöl, Lebertran, Ethern, wie z. B. Diethylenglykoldimethylether, oder auch Polyethern, z. B. Polyethylenglykol, oder auch in Gegenwart anderer pharmakologisch unbedenklicher Polymerträger, wie z. B. Polyvinylpyrrolidon, oder anderen pharmazeutisch annehmbaren Zusatzstoffen wie Stärke, Cyclodextrin oder Polysacchariden. Ferner können die erfindungsgemäßen Verbindungen in Kombination mit anderen Arzneistoffe, gegeben werden.

Die Verbindungen der Formel I werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 5000 mg, vorzugsweise jedoch in Dosisbereich 10 bis 1000 mg.

In den nachfolgenden Beispielen sind die jeweils berechneten Monoisotop-Molmassen angegeben.

Massenspektren wurden soweit nicht anders angegeben, mit FAB-Technik unter Zusatz von LiCl und 3-Nitrobenzaldehyd[3-NBA] aufgenommen.

Ausgangsverbindungen, die die Gallensäurestruktur aufweisen, sind z. T. bereits beschrieben worden (vergl. z. B. EP-A-0 417 725, EP-A-0 489 423 und EP-A-0 548 793).

$R^1$ ist im Beispiel 6 definiert.

Folgende Beispiele 1-19, 24, 25 und 28-61 beziehen sich auf die Herstellung von Verbindungen, die nicht zum beanspruchten Gegenstand der Erfindung gehören. Diese Beispiele erleichtern aber das Verständnis der Herstellung der Verbindungen der Erfindung (insbesondere die Verbindungen der Beispiele 20-23 und 26-27).

Beispiel 1

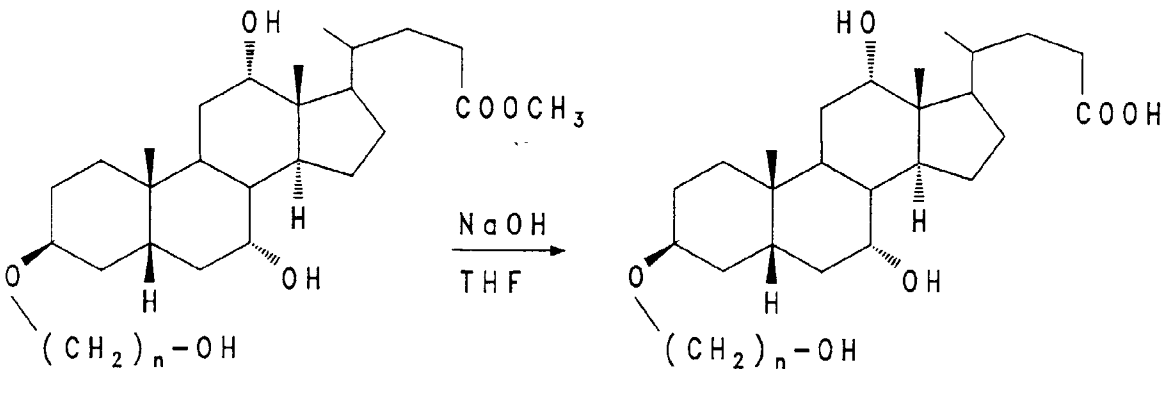


a

n = 5

1 g (1,96 mmol) des Methylesters a wird in 15 ml Tetrahydrofuran (THF) oder 1,4-Dioxan gelöst und mit 10 ml 2N NaOH intensiv über Nacht bei Raumtemperatur gerührt. Danach verdünnt man mit viel Wasser und säuert unter Eiskühlung mit halbkonzentrierter Salzsäure an. Die Fällung wird durch 1 Stunde Nachrühren unter Eiskühlung vervollständigt, der gebildete Niederschlag wird abgesaugt und mit kaltem Wasser nachgewaschen. Nach Umkristallisation aus Ethanol/Wasser und Trocknen im Vakuum erhält man 940 mg (96 %) Beispiel 1.

$C_{29}H_{50}O_6$ (494)     MS: 501 (M + $Li^+$).

Die folgenden Beispiele 2 bis 7 werden analog zu "Beispiel 1" aus den entsprechenden Gallensäureresten hergestellt:

| Beispiel Nr. | wie "Beispiel 1" mit n = | Summenformel | MW | MS |
|---|---|---|---|---|
| 2 | 6 | $C_{30}H_{52}O_6$ | 508 | 515 (M+$Li^+$) |
| 3 | 8 | $C_{32}H_{56}O_6$ | 536 | 543 (M+$Li^+$) |
| 4 | 9 | $C_{33}H_{58}O_6$ | 550 | 557 (M+$Li^+$) |
| 5 | 10 | $C_{34}H_{60}O_6$ | 564 | 571 (M+$Li^+$) |

Beispiel 6

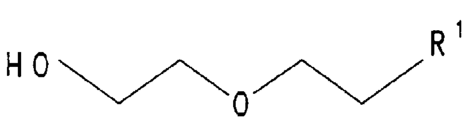

$R^1$ =

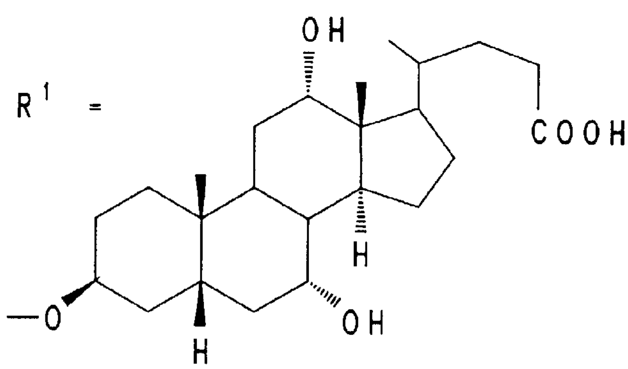

$C_{28}H_{48}O_7$ (496) MS: 503 (M + $Li^+$)

Beispiel 7

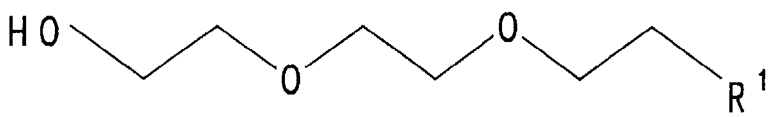

$C_{30}H_{52}O_8$ (540) MS: 547 (M + $Li^+$)

Beispiel 8

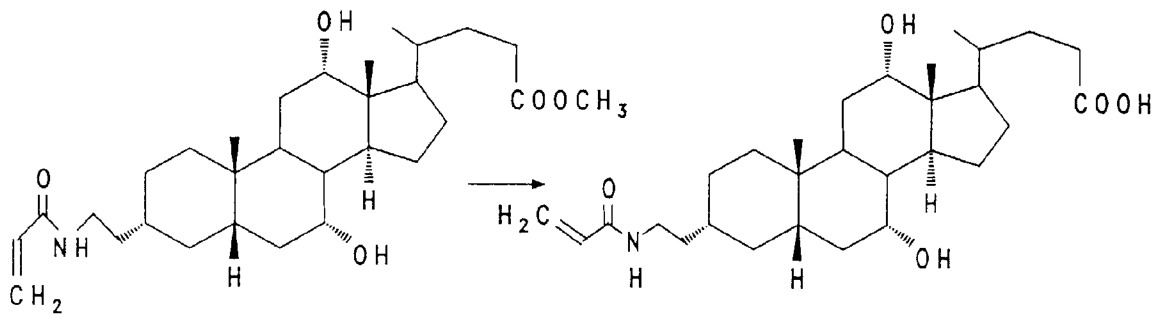

100 mg (0,2 mmol) Methylester werden in 10 ml Dioxan gelöst und mit 3 ml halbkonz. Natronlauge 6 Stunden bei Raumtemperatur gerührt. Man verdünnt mit Wasser, säuert mit halbkonz. Salzsäure an und erhält nach Absaugen und Waschen die Säure "Beispiel 8" (50 mg, 51 %).

$C_{29}H_{47}NO_5$ (489) MS: 496 (M + $Li^+$)

Folgende Beispielsubstanzen wurden wie "Beispiel 8" hergestellt:

Beispiel 9

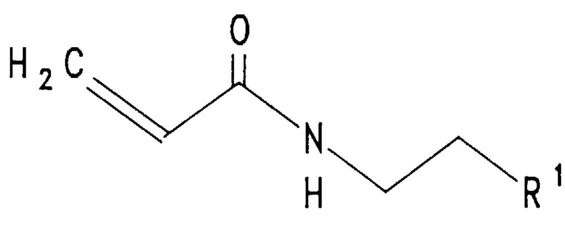

$C_{29}H_{47}NO_6$ (505) MS: 512 (M + $Li^+$)

Beispiel 10

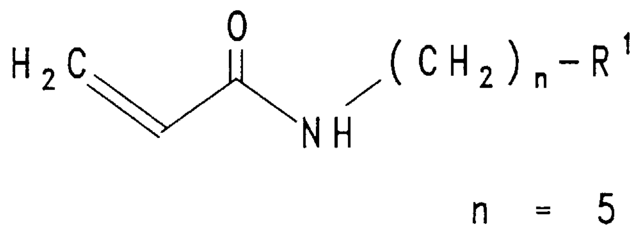

$C_{32}H_{53}NO_6$ (547) MS: 554 (M + Li+)

Beispiel 11

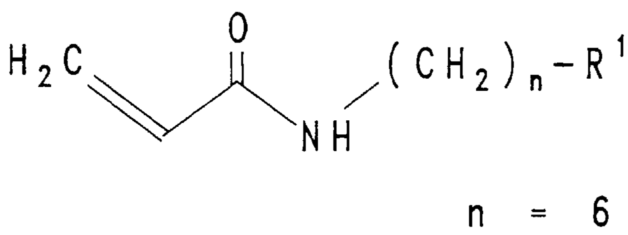

$C_{33}H_{55}NO_6$ (561) MS: 568 (M + Li+)

Beispiel 12

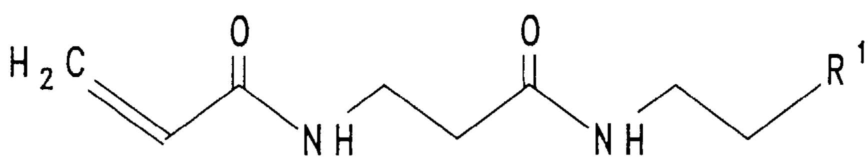

$C_{32}H_{52}N_2O_7$ (576) MS: 583 (M + Li+)

Beispiel 13

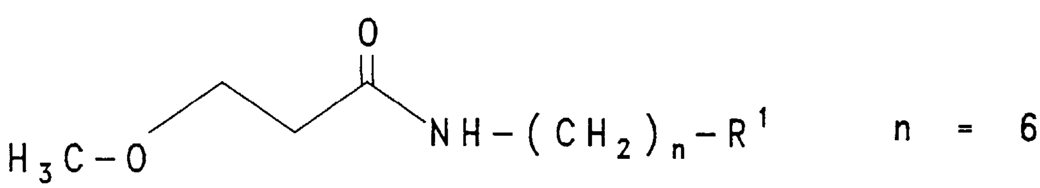

$C_{34}H_{59}NO_7$ (593) MS: 600 (M + $Li^+$)

Beispiel 14

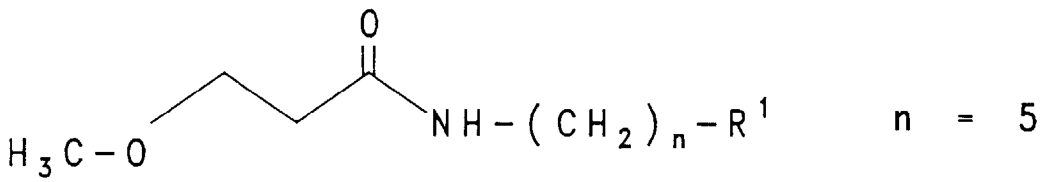

$C_{33}H_{57}NO_7$ (579) MS: 586 (M + $Li^+$)

Beispiel 15

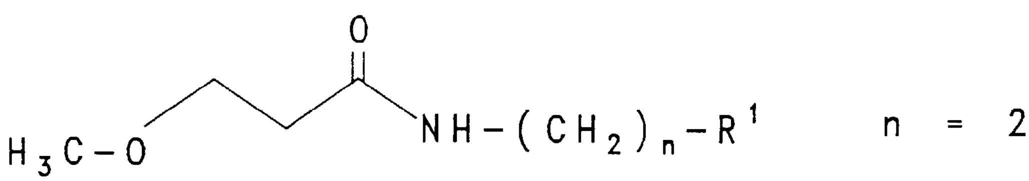

$C_{30}H_{51}NO_7$ (537) MS: 544 (M + $Li^+$)

Beispiel 16

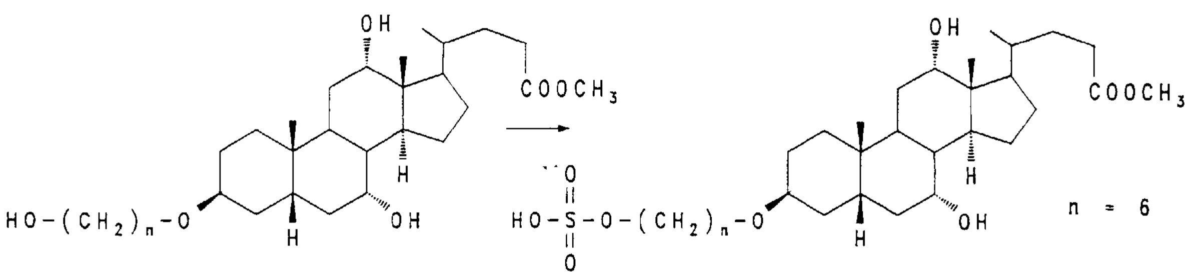

3,14 g (6 mmol) des primären Alkohols a (n = 6) werden in 100 ml trockenem Dichlormethan mit 0,84 ml Triethylamin versetzt und auf -10°C gekühlt. Bei dieser Temperatur gibt man 0,4 ml (6 mmol) Chlorsulfonsäure in 20 ml trockenem Dichlormethan zur Lösung. Nach 1 Stunde bei 0°C und 1 Stunde bei Raumtemperatur wird mit Wasser versetzt, die organische Phase wird abgetrennt, die wäßrige Phase mehrfach mit Ethylacetat extrahiert und die vereinigten organischen Phasen werden getrocknet und eingeengt. Der Rückstand wird chromatographisch ($SiO_2$, Ethylacetat/Methanol = 3:1) gereinigt. Man erhält 1,45 g (40 %) "Beispiel 16".

$C_{31}H_{54}O_9S$ (602) MS: 631 (M-$H^+$ + $Li^+$ +$Na^+$)
615 (M-$H^+$ +2$Li^+$)

Beispiel 17

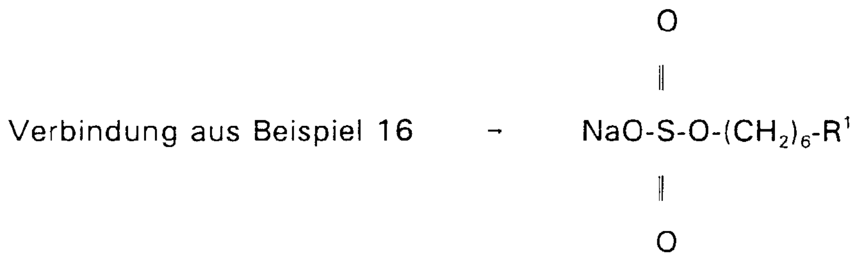


0,5 g (0,83 mmol) "Beispiel 16" werden in 20 ml Dioxan mit 7 ml halbkonz. Natronlauge 6 Stunden bei Raumtemperatur gerührt. Anschließend wird unter Kühlung mit halbkonz. Salzsäure angesäuert und im Vakuum eingeengt. Der Rückstand wird durch Säulenfiltration ($SiO_2$, Ethylacetat/Methanol = 3:1) gereinigt. Man erhält 254 mg (52 %) "Beispiel 17".

$C_{30}H_{51}NaO_9S$ (610) MS: 617 (M + $Li^+$)
601 (M-$Na^+$+2$Li^+$)

Beispiel 18

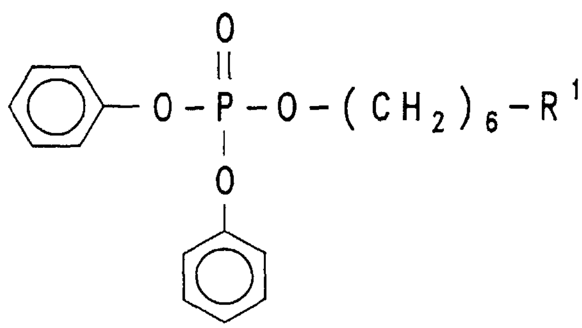

Zu einer Lösung von 2,6 g (5,12 mmol) "Beispiel 2" in 20 ml Pyridin gibt man tropfenweise bei 0 bis 5°C 15 ml Phosphorsäurediphenylesterchlorid und rührt 2 Stunden bei Raumtemperatur nach. Man gießt auf 200 ml Eiswasser, versetzt unter Rühren und Kühlung mit ca. 15 ml konzentrierter Schwefelsäure und extrahiert mehrfach mit Ethylacetat. Die organische Phase wird getrocknet, eingeengt und der Rückstand wird chromatographisch gereinigt ($SiO_2$, $CH_2Cl_2$/$CH_3OH$ = 10:1). Man erhält 1,78 g (47 %) "Beispiel 18".

$C_{42}H_{61}O_9P$ (740) MS: 747 (M + $Li^+$)

Beispiel 19

O

‖

HO-P-O-$(CH_2)_6$-$R^1$

|

OH

1 g (1,35 mmol) "Beispiel 18" wird in 50 ml Eisessig mit einer Spatelspitze Platin auf Kohle in einer Schüttelente hydriert. Nach beendeter Umsetzung (ca. 4 Stunden) wird vom Katalysator abgesaugt und eingeengt. Der Rückstand wird durch Säulenfiltration gereinigt ($SiO_2$, Ethylacetat/$CH_3OH$ = 2:1). Man erhält 270 mg (34 %) "Beispiel 19".

$C_{30}H_{53}O_9P$ (588) MS: 601 (M-$H^+$ + $2Li^+$)
595 (M + $Li^+$)

Beispiel 20

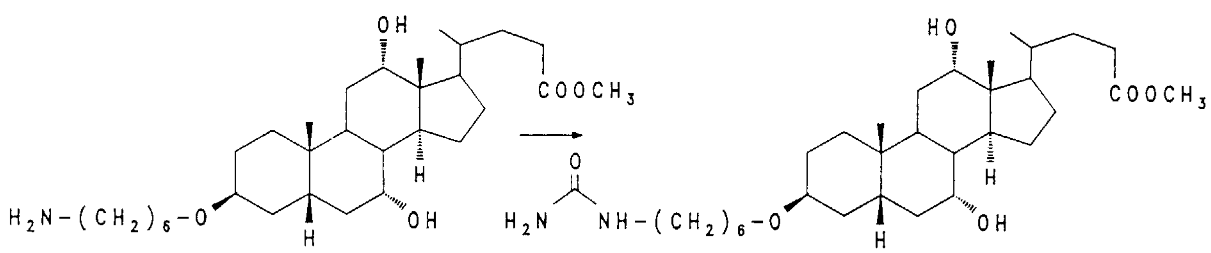

b

2,24 g (4 mmol) Amin b und 324 mg (4 mmol) Kaliumcyanat werden in 60 ml Wasser suspendiert und zum Sieden erhitzt. Es entsteht eine Lösung aus der sich nach kurzer Zeit ein Feststoff abscheidet. Man rührt 30 Minuten bei Siedetemperatur, setzt nach dem Abkühlen ca. 40 ml Wasser zu und säuert mit verdünnter Salzsäure an. Man extrahiert mehrfach mit Ethylacetat, trocknet die organische Phase, engt im Vakuum ein und reinigt den Rückstand chromatographisch ($SiO_2$, EtOAC/$CH_3OH$ = 10:1). Man erhält 520 mg (23 %) "Beispiel 20".

$C_{32}H_{56}N_2O_6$ (564) MS: 571 (M + $Li^+$)

Beispiel 21

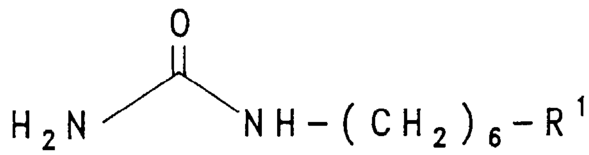

450 mg (0,8 mmol) "Beispiel 20" werden in 10 ml Dioxan mit 5 ml halbkonzentrierter Natronlauge 6 Stunden bei Raumtemperatur gerührt. Nach beendeter Umsetzung wird mit Wasser verdünnt, mit Salzsäure angesäuert und 1 Stunde im Eisbad nachgerührt. Man saugt ab, wäscht mit Wasser nach und erhält nach dem Trocknen im Vakuum 430 mg (97 %) "Beispiel 21".

$C_{31}H_{54}N_2O_6$ (550) MS: 557 (M + $Li^+$)

Beispiel 22

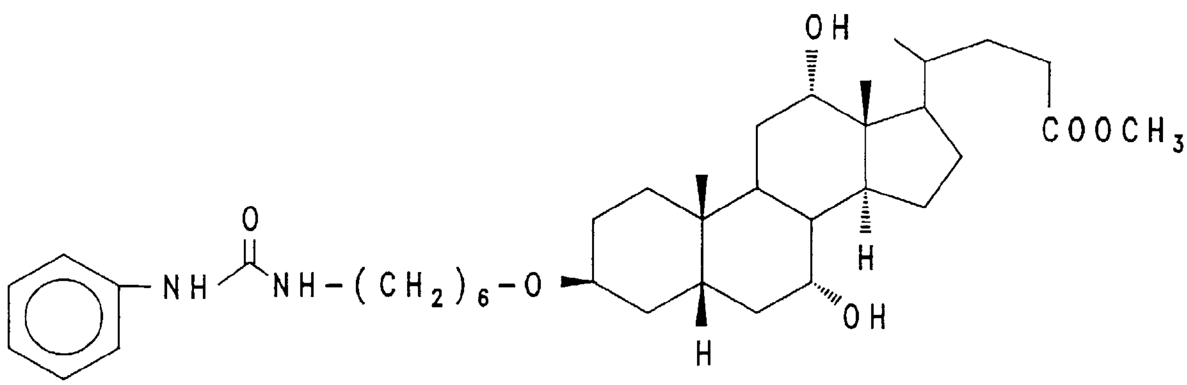

1,04 g (2 mmol) Amin b (Beispiel 20) werden in 50 ml trockenem Dichlormethan und 28 ml Triethylamin bei 0°C mit 2 mmol Phenylisocyanat in 5 ml Dichlormethan versetzt. Man rührt 6 Stunden bei Raumtemperatur nach und arbeitet wie unter "Beispiel 16" beschrieben unter Ansäuern der wäßrigen Phase auf. Nach Säulenfiltration ($CH_2Cl_2/CH_3OH$ = 10:1) erhält man 6540 mg (51 %) "Beispiel 22".

$C_{38}H_{60}N_2O_6$ (640) MS: 647 (M + $Li^+$)

Beispiel 23

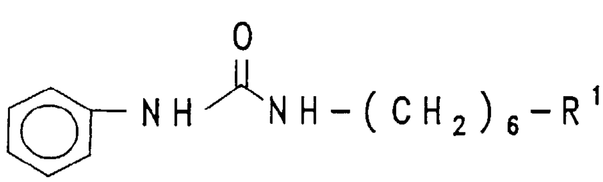

$C_{37}H_{58}N_2O_6$ (626)   MS: 633 ($M + Li^+$)

Beispiel 24

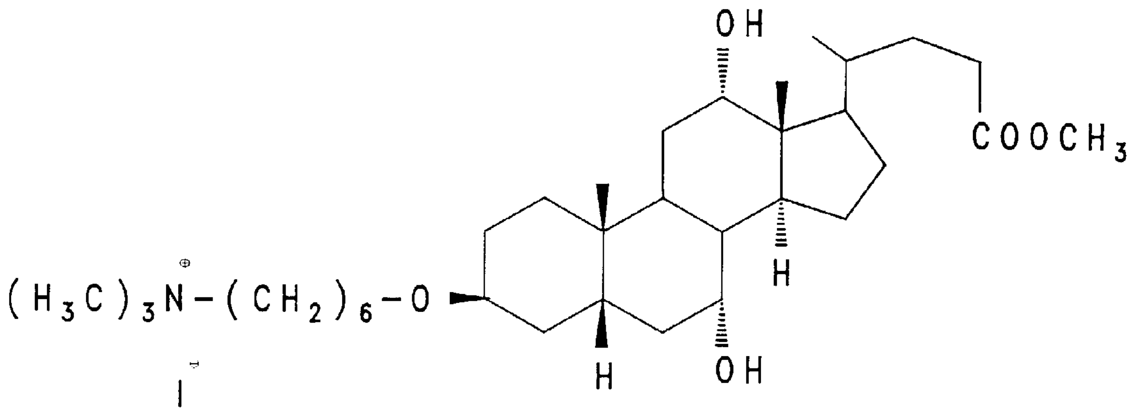

2,08 g (4 mmol) Amin b, 10 ml Triisobutylamin und 5 ml Jodmethan werden in 50 ml Acetonitril 2 Stunden zum Sieden erhitzt. Man entfernt alle flüchtigen Bestandteile im Vakuum und reinigt den Rückstand chromatographisch ($SiO_2$, $CH_2Cl_2/CH_3OH$ = 10:1). Man erhält 1,2 g (43 %) "Beispiel 24".

$C_{34}H_{62}INO_5$ (691)   MS (FAB, 3-NBA): 564 ($M-I^{\ominus}$)

Beispiel 25

$$(H_3C)_3N^{\oplus}\text{-}(CH_2)_6\text{-}R^1$$
$$Cl^{\ominus}$$

Aus Beispiel 24 wird analog zu "Beispiel 21" Verbindung Beispiel 25 hergestellt. Die Reinigung des Rohprodukts erfolgt durch Mitteldruckchromatographie an RP-8-Kieselgel ($CH_3OH/H_2O$ = 7:3).

$C_{33}H_{60}ClNO_5$ (585)   MS (FAB, 3-NBA): 550 ($M-Cl^{\ominus}$)

Beispiel 26

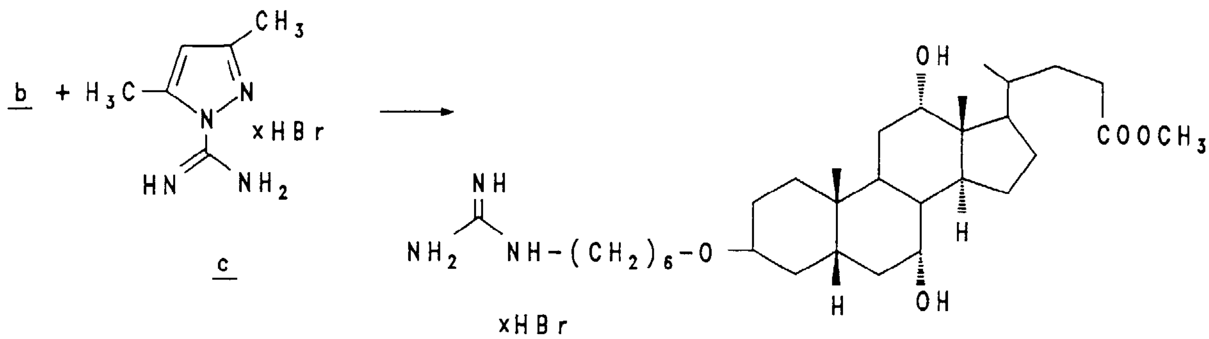

1,04 g (2 mmol) Amin b und 276 mg (2 mmol) Pyrazol c werden in 40 ml trockenem Acetonitril 10 Stunden zum Rückfluß erhitzt. Nach Abkühlung und Zusatz von Ether bildet sich ein Niederschlag, der abgesaugt und mit trockenem Ether nachgewaschen wird. Nach dem Trocknen erhält man 450 mg "Beispiel 26".

$C_{32}H_{58}BrN_3O_5$ (643)  MS: 570 (M-HBr + $Li^+$)
564 (M-$Br^\ominus$)

Beispiel 27

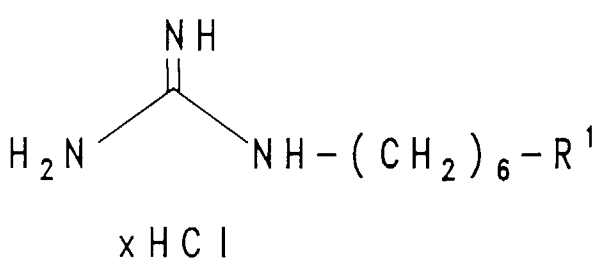

wird analog zu "Beispiel 21" hergestellt.

$C_{31}H_{56}ClN_3O_5$ (585)  MS: 556 (M-HCl + $Li^+$)
550 (M-$Cl^\ominus$)

Beispiel 28

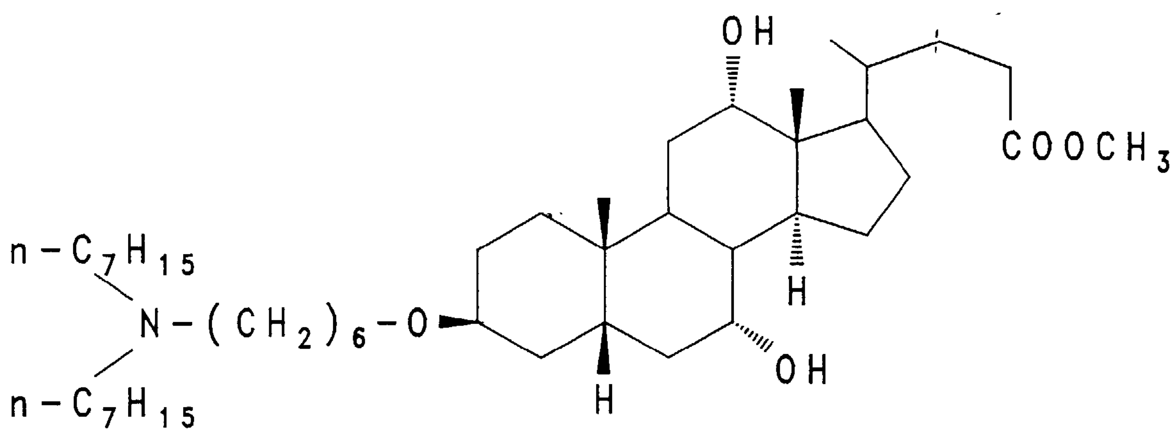

1,0 g (1,9 mmol) Amin b, 265 mg $NaBH_3CN$ und 610 mg Heptanal werden in 10 ml trockenem Methanol 48 Stunden bei Raumtemperatur gerührt. Man engt im Vakuum ein, verteilt den Rückstand zwischen Essigester und ges. Hydrogencarbonat-Lösung und reinigt den Rückstand der organischen Phase chromatographisch. Neben einer geringen Menge Monoheptylaminoderivat erhält man 650 mg (49 %) "Beispiel 28".

$C_{45}H_{83}NO_5$ (718)    MS: 725 (M + $Li^+$)

Beispiel 29

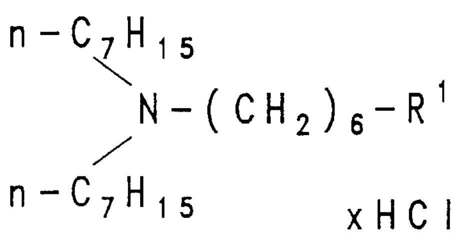

wird analog zu "Beispiel 21" hergestellt. Vom öligen Rohprodukt nach dem Ansäuern wird die wäßrige Phase dekantiert und der Rückstand mit Ethylacetat ausgerührt, danach abgesaugt und getrocknet.

$C_{44}H_{82}ClNO_5$ (740)    MS: 711 (M-HCl + $Li^+$)
705 (M-$Cl^{\ominus}$)

Beispiel 30

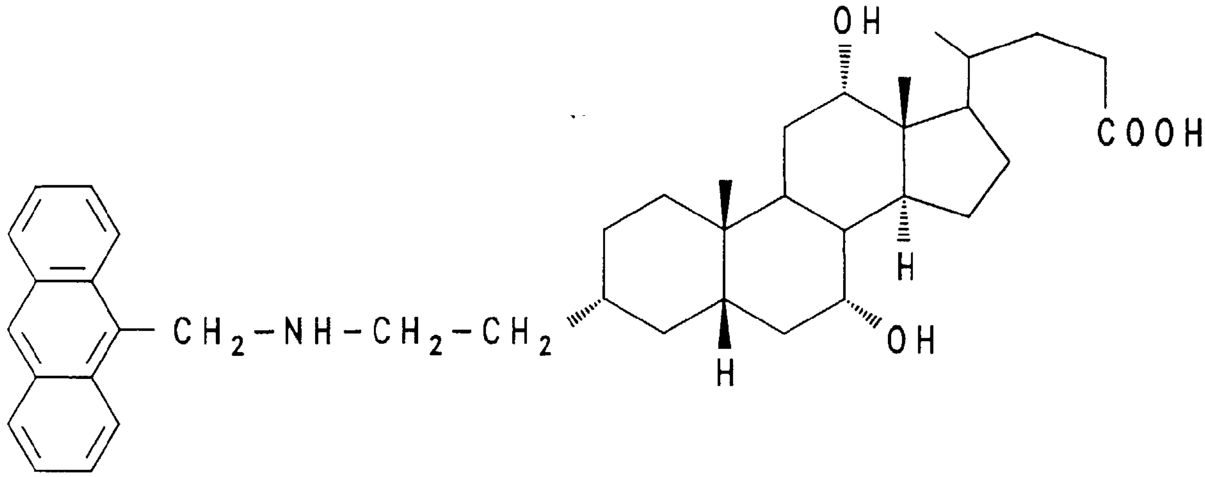

wird analog zu "Beispiel 28" und "Beispiel 29" durch reduktive Aminierung von Anthracen-9-carbaldehyd mit 3α-(Aminoethyl)-7α,12α-dihydroxy-24-cholansäuremethylester (d) und anschließende alkalische Verseifung hergestellt.

$C_{41}H_{55}NO_4$ (625)  MS: 632 (M + $Li^+$)

Beispiel 31

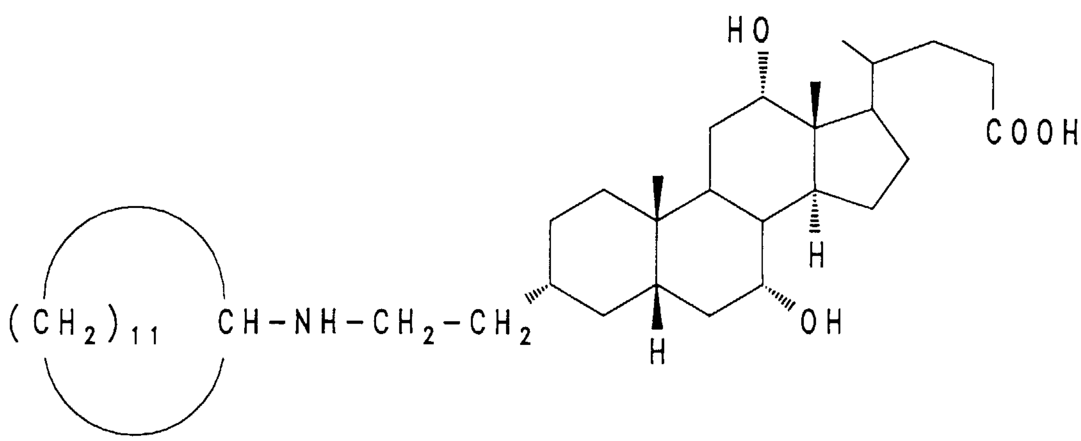

wird analog zu "Beispiel 30" unter Verwendung von Cyclododecanon als Carbonylkomponente hergestellt.

$C_{38}H_{87}NO_4$ (602)  MS: 609 (M+$Li^+$)

Beispiel 32

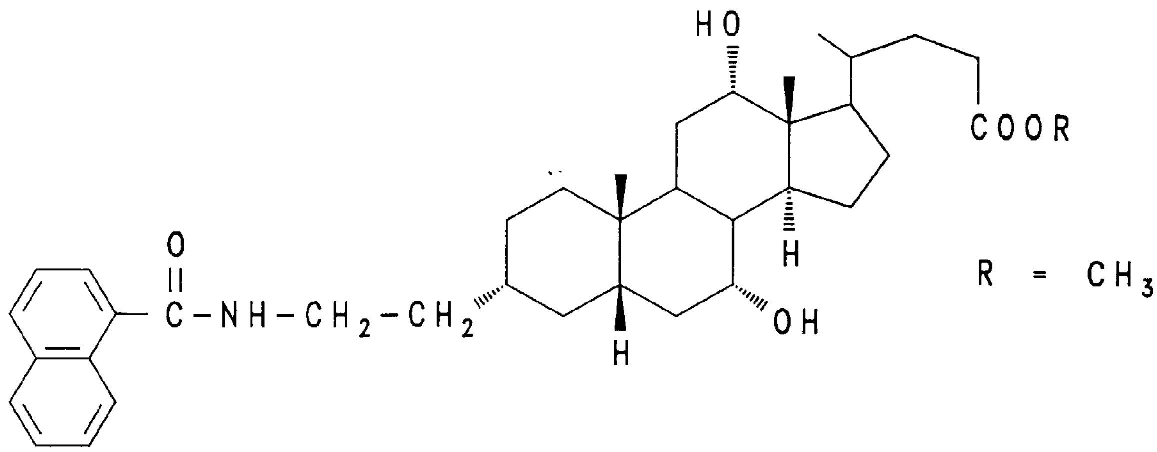

0,9 g (2 mmol) Amin d und 0,6 ml Triethylamin in 20 ml trockenem $CH_2Cl_2$ werden unter Eiskühlung mit 0,38 g (2 mmol) Naphthoylchlorid in 5 ml $CH_2Cl_2$ versetzt. Das Gemisch wird 1 Stunde bei 0°C nachgerührt und über Nacht stehengelassen. Man versetzt mit Wasser, säuert an und extrahiert mehrfach mit $CH_2Cl_2$. Der Rückstand der organischen Phase wird chromatographisch gereinigt ($SiO_2$, EtOAc/Cyclohexan = 3:1). Man erhält 1 g (83 %) "Beispiel 32".

$C_{38}H_{53}NO_5$ (603) MS: 610 (M + $Li^+$)

Beispiel 33

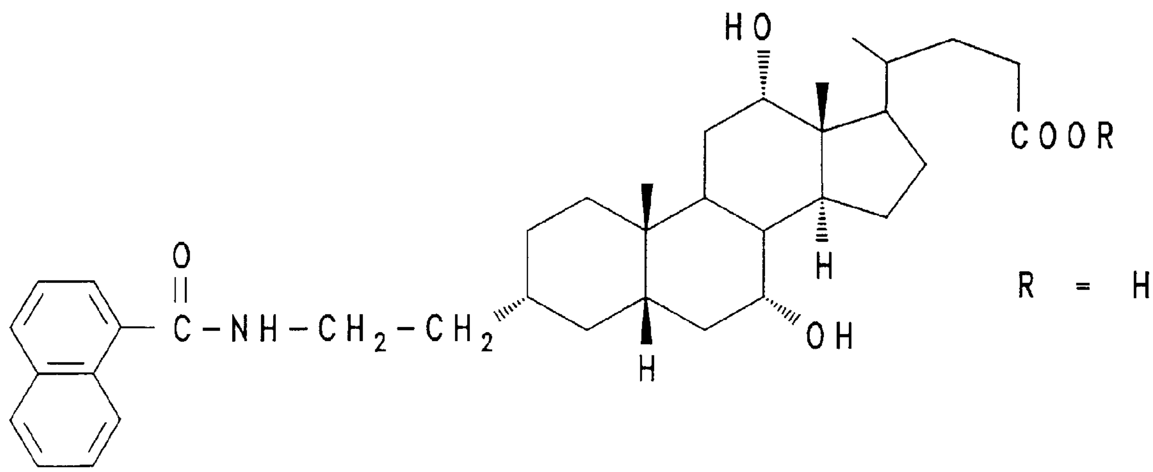

wird analog "Beispiel 21" hergestellt.

$C_{37}H_{51}NO_5$ (589) MS: 596 (M + $Li^+$)

Beispiel 34

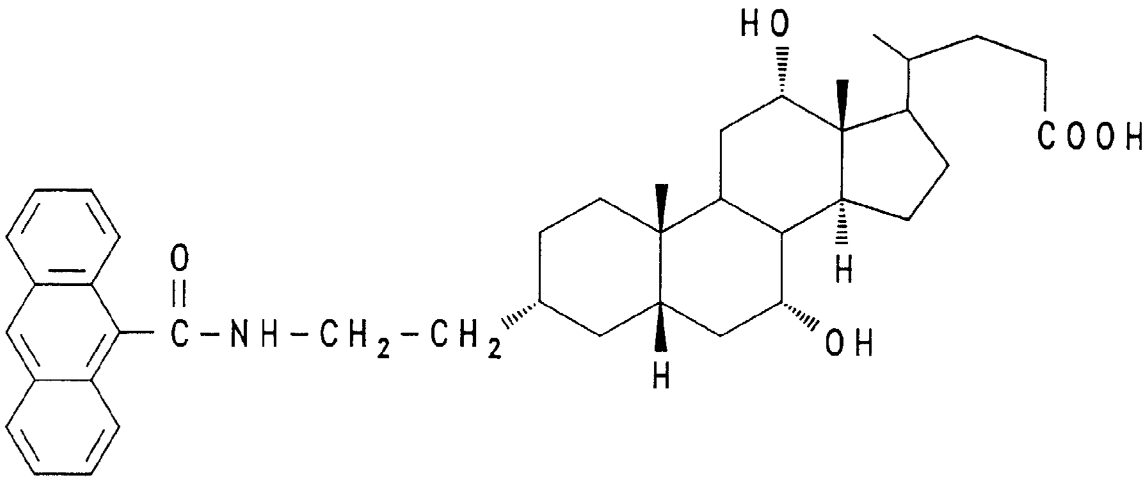

wird analog zu "Beispiel 32" und "Beispiel 33" unter Verwendung von Anthracen-9-Carbonsäurechlorid hergestellt.

$C_{41}H_{53}NO_5$ (639) MS: 646 (M + $Li^+$)

Beispiel 35

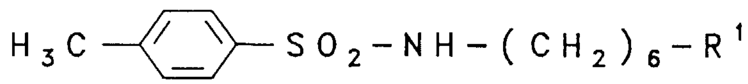

wird analog "Beispiel 34" unter Verwendung von p-Toluol-sulfonsäurechlorid und Amin b hergestellt.

$C_{37}H_{59}NO_7S$ (661) MS: 668 (M + $Li^+$)

Beispiel 36

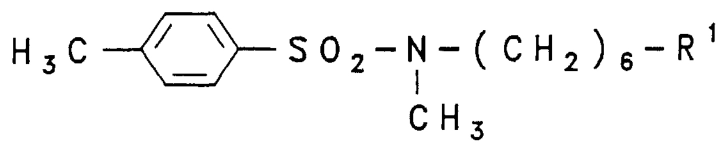

wird analog zu "Beispiel 35" hergestellt. Der als Zwischenprodukt erhaltene Methylester wird in Dimethylformamid nach Deprotonierung durch Natriumhydrid mit Jodmethan bei Raumtemperatur methyliert. Anschließend wird analog "Beispiel 35" alkalisch verseift.

$C_{38}H_{61}NO_7S$ (675) MS: 688 (M-$H^+$ + 2$Li^+$)
682 (M + $Li^+$)

Beispiel 37

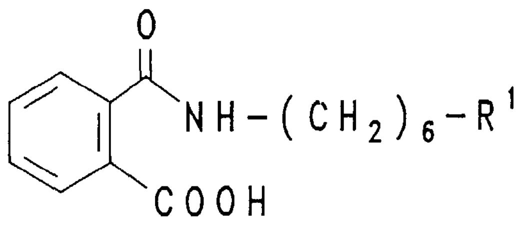

wird analog zu "Beispiel 34" unter Verwendung von o-Phthalsäureanhydrid und Amin b hergestellt.

$C_{38}H_{57}NO_8$ (655) MS: 668 ($M-H^+ + 2Li^+$)
662 ($M + Li^+$)

Beispiel 38

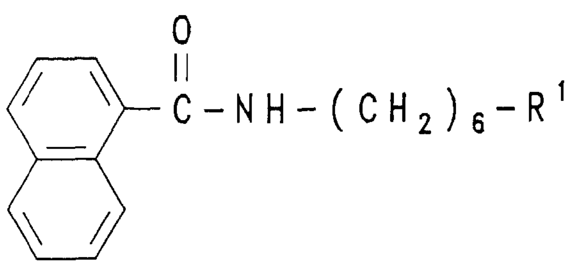

wird analog zu "Beispiel 32" / "Beispiel 33" unter Verwendung von Amin b hergestellt.

$C_{41}H_{59}NO_6$ (661) MS: 668 ($M + Li^+$)

Beispiel 39

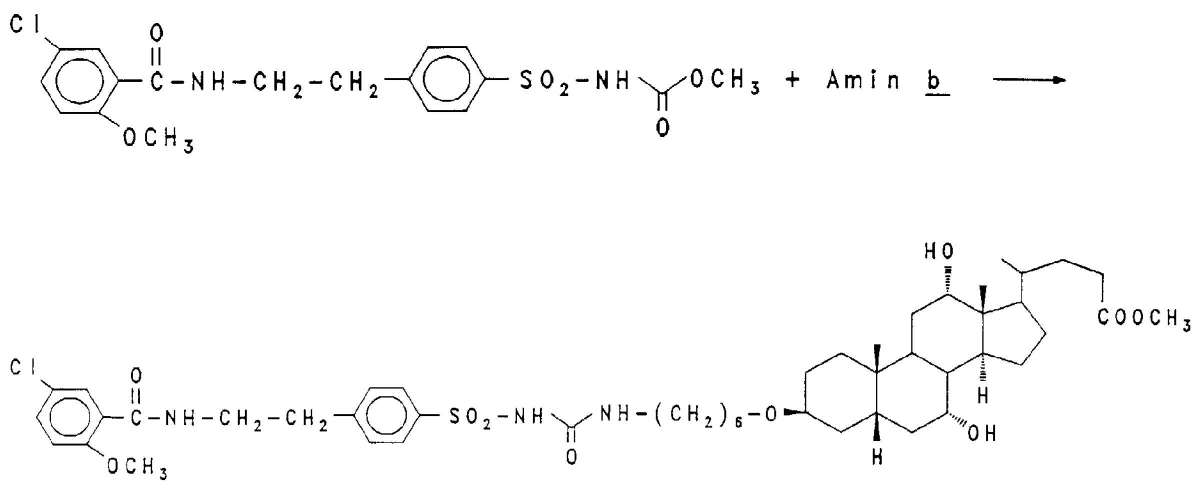

426 mg (1 mmol) Urethan und 782 mg (15 mmol) Amin b werden in 50 ml Dioxan 4 Stunden zum Rückfluß erhitzt. Danach wird eingeengt und der Rückstand chromatographisch gereinigt ($SiO_2$, $CH_2Cl_2/CH_3OH$ = 10:1). Man erhält 540 g (59 %) "Beispiel 39".

$C_{48}H_{70}ClN_3O_{10}S$ (915)      MS: 922 (M + $Li^+$)

Beispiel 40

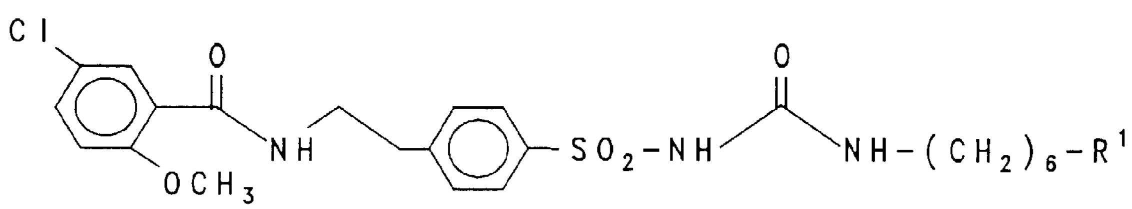

wird analog "Beispiel 21" hergestellt.

$C_{47}H_{68}ClN_3O_{10}S$ (901)      MS (Elektrospray): 902 (M + $H^+$)

Beispiel 41

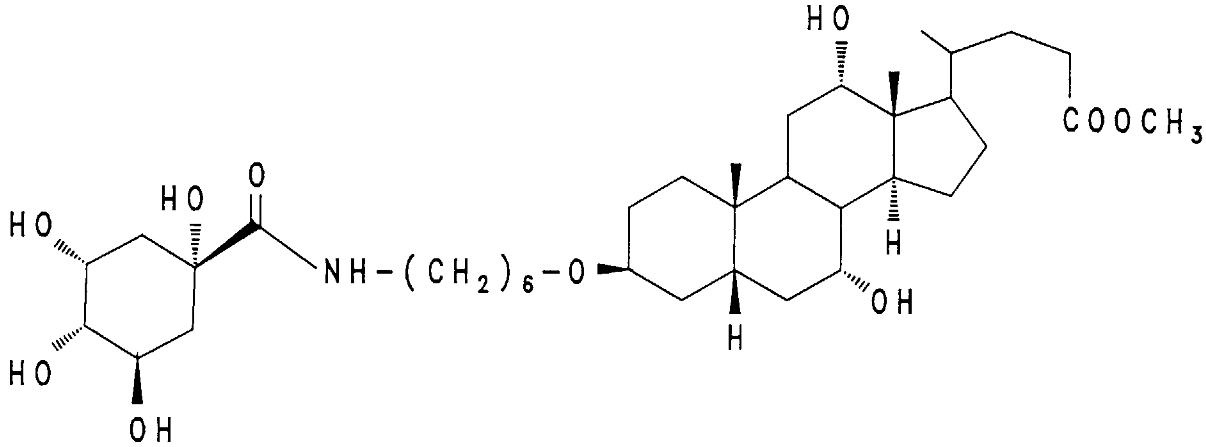

Zu einer Lösung von 1,56 g (3 mmol) Amin b, 576 mg (3 mmol) Chinasäure und 490 mg (83,6 mmol) Hydroxybenzotriazol in 100 ml THF werden 750 mg (3,6 mmol) Dicyclohexylcarbodiimid gegeben. Man rührt 40 Stunden bei Raumtemperatur. Man filtriert vom gebildeten Harnstoff ab, engt die Lösung ein und nimmt den Rückstand mit Ethylacetat auf. Die Lösung wird mit ges. $NaHCO_3$-Lösung, 2N Citronensäure, ges. $NaHCO_3$-Lösung und Wasser gewaschen. Der Rückstand der organischen Phase wird chromatographisch gereinigt ($SiO_2$, Ethylacetat/$CH_3OH$ = 5:1). Man erhält 1,2 g (58 %) "Beispiel 41".

$C_{38}H_{65}NO_{10}$ (695) MS: 702 (M + $Li^+$)

Beispiel 42

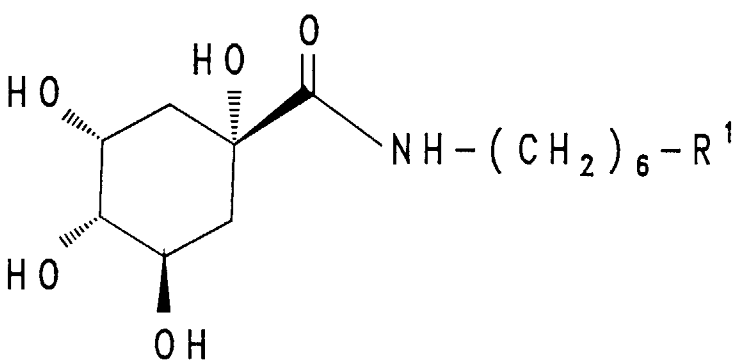

wird analog "Beispiel 21" hergestellt.

$C_{37}H_{63}NO_{10}$ (681) MS (FAB, 3-NBA): 682 (M + $H^+$)

Beispiel 43

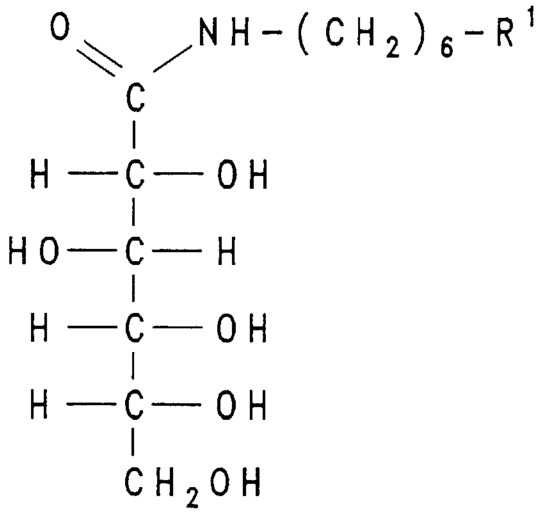

wird analog zu "Beispiel 41" / "Beispiel 42" unter Verwendung von Gluconsäure hergestellt.

$C_{36}H_{63}NO_{11}$ (685)  MS: 714 ($M-H^+ + Li^+ + Na^+$)

Beispiel 44

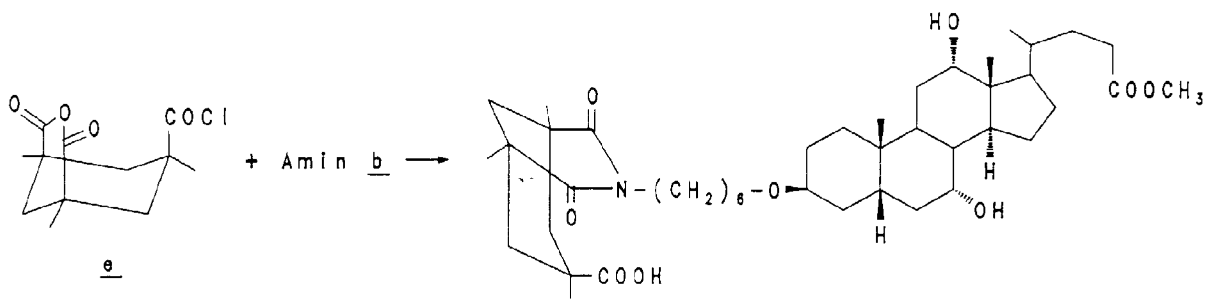


1,04 g (4 mmol) Säurechlorid e, 2,1 g (4 mmol) Amin b und eine Spatelspitze 4-Dimethylaminopyridin werden in 40 ml trockenem Pyridin 6 Stunden bei Raumtemperatur gerührt. Nach Stehen über Nacht bei Raumtemperatur wird im Vakuum eingeengt. "Beispiel 44" wird nach chromatographischer Reinigung isoliert ($SiO_2$, $CH_2Cl_2/CH_3OH$ = 20:1).

$C_{43}H_{69}NO_9$ (743)  MS: 750 ($M + Li^+$)

Beispiel 45

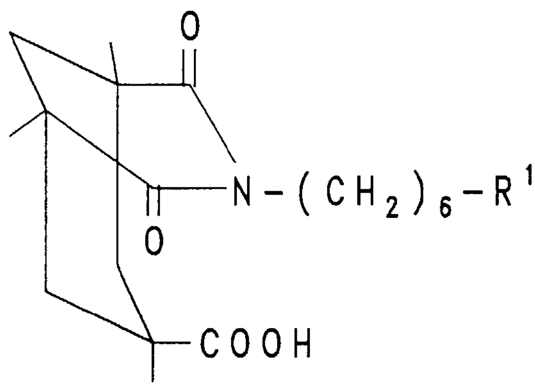

wird analog zu "Beispiel 21" hergestellt.

$C_{42}H_{67}NO_9$ (729) MS: 742 ($M-H^+ + 2Li^+$)
736 ($M + Li^+$)

Beispiel 46

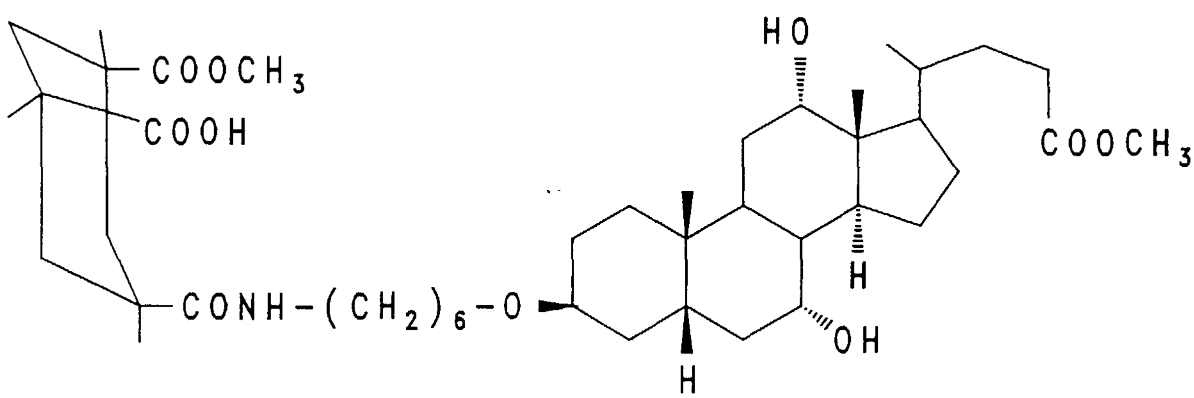

1,3 g (5 mmol) Säurechlorid e und 0,8 ml Triethylamin in 50 ml trockenen $CH_2Cl_2$ werden unter Eiskühlung mit 2,6 g (5 mmol) Amin b in $CH_2Cl_2$ versetzt und 1 Stunde bei 0°C gerührt. Danach versetzt man mit einem Überschuß Methanol, läßt auf Raumtemperatur kommen, setzt Wasser zu und säuert mit verdünnter Salzsäure an. Die wäßrige Phase wird mehrfach mit $CH_2Cl_2$ ausgeschüttelt. Nach chromatographischer Reinigung des Rückstands der organischen Phase ($SiO_2$, $CH_2Cl_2/CH_3OH$ = 10:1) erhält man "Beispiel 46".

$C_{44}H_{73}NO_{10}$ (775) MS: 783 ($M + Li^+$)

Beispiel 47

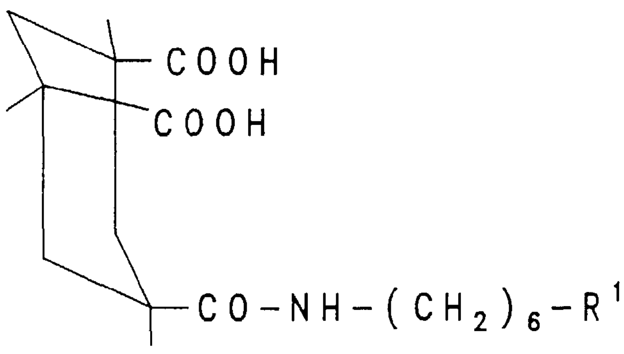

wird analog zu "Beispiel 21" hergestellt.

$C_{42}H_{69}NO_{10}$ (747) MS: 760 ($M-H^+ + 2Li^+$)
754 ($M + Li^+$)

Beispiel 48

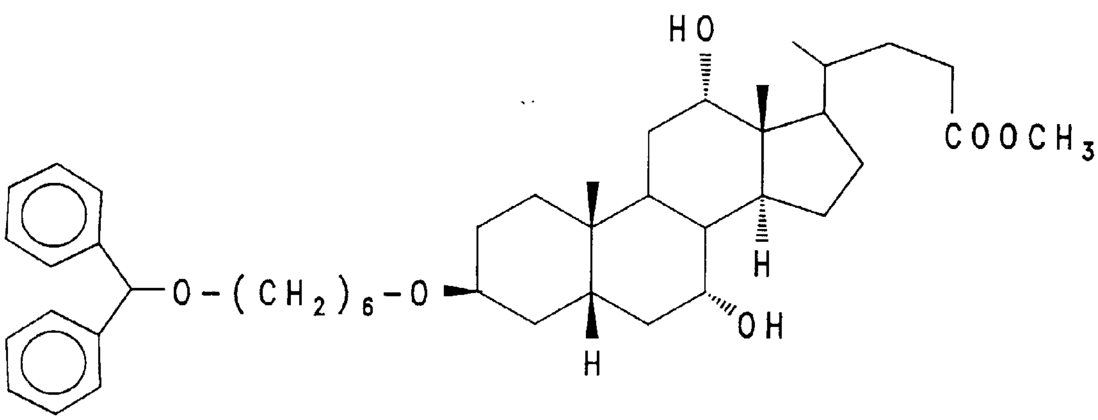

3,14 g (6 mmol) Alkohol a (n = 6) werden mit 3 ml Ethyldiisopropylamin und 1,5 g Diphenylmethylbromid in 50 ml DMF 8 Stunden auf 100°C erwärmt. Man erhält "Beispiel 48" nach wäßriger Aufarbeitung und chromatographischer Reinigung ($SiO_2$, $CH_2Cl_2/CH_3OH$ = 10:1).

$C_{44}H_{64}O_6$ (688) MS: 695 ($M + Li^+$)

Beispiel 49

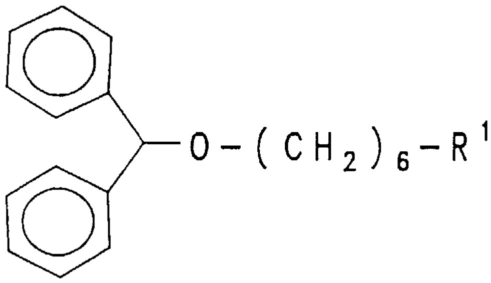

wird analog zu "Beispiel 21" hergestellt.

$C_{43}H_{62}O_6$ (674)  MS: 681 (M + $Li^+$)

Analog Beispiel 1 werden die folgenden Verbindungen 50 bis 52 aus den entsprechenden Gallensäureestern durch alkalische Esterverseifung hergestellt:

Beispiel 50

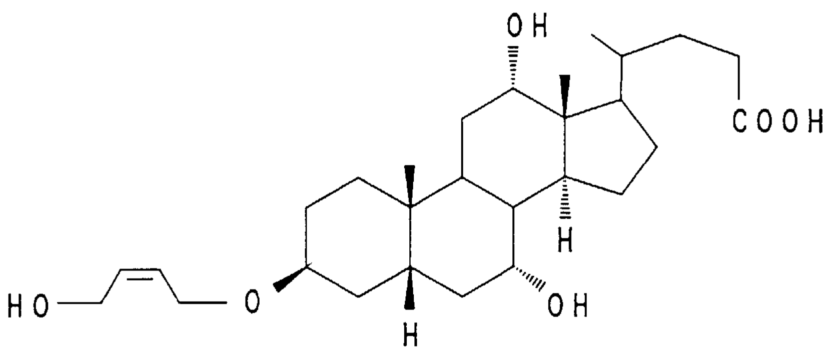

$C_{28}H_{46}O_6$  MW: 478  MS: 485 (M + $Li^+$)

Beispiel 51

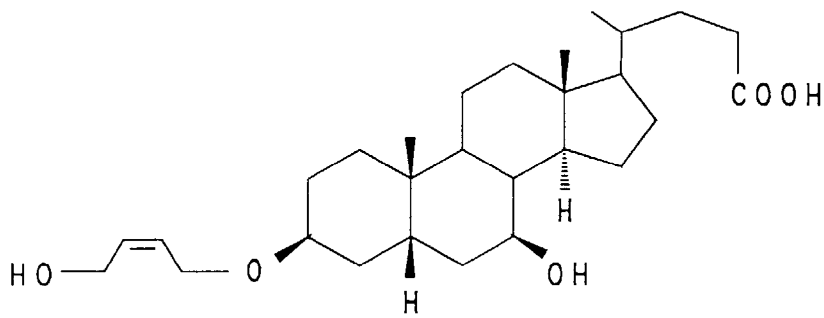

$C_{28}H_{46}O_5$ MW: 462 MS: 469 (M + $Li^+$)

Beispiel 52

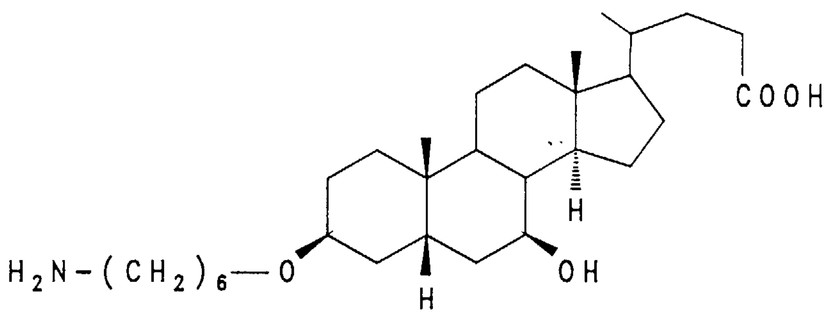

$C_{30}H_{53}NO_4$ MW: 491 MS: 498 (M + $H^+$)

Beispiel 53

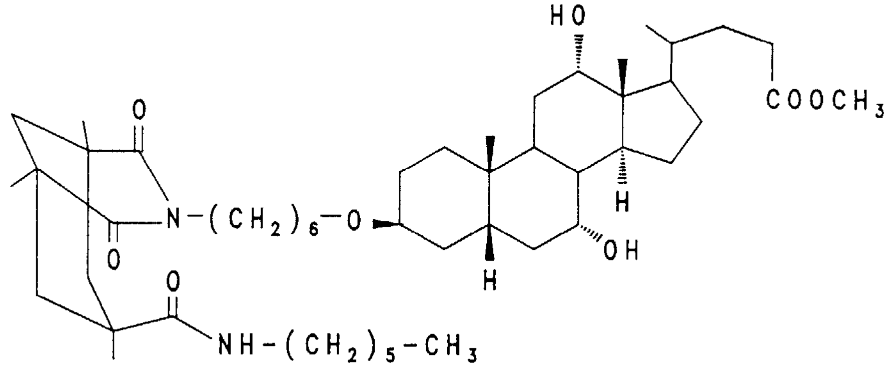

wird aus Beispiel 44 und n-Hexylamin analog zu Beispiel 41 mit einer Reaktionszeitvon 25 Stunden hergestelltn.

$C_{49}H_{82}N_2O_8$ (827)  MS: 834 (M + $Li^+$)

Beispiel 54

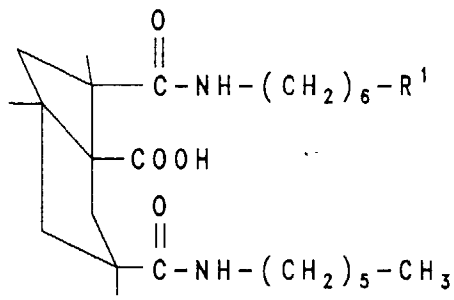

170 mg "Beispiel 53" werden in 5 ml Dioxan gelöst, mit 1,5 ml halbkonzentrierter Natronlauge und 25 ml Wasser versetzt und 12 Stunden bei Raumtemperatur gerührt.
Eine Trübung wird abfiltriert, das Filtrat wird mit verdünnter Salzsäure angesäuert, 1 Stunde nachgerührt und der gebildete Niederschlag wird abgesaugt. Man erhält nach dem Trocknen 154 mg "Beispiel 54".

$C_{48}H_{82}N_2O_9$ (831)  MS: 838 (M + $Li^+$)

Beispiel 55

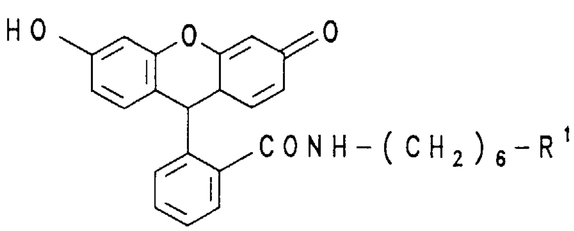

wird analog zu "Beispiel 53" und "Beispiel 54" aus Fluorescein und Amin b hergestellt.

$C_{50}H_{63}NO_9$ (821)  MS: 828 (M + $Li^+$)

Beispiel 56

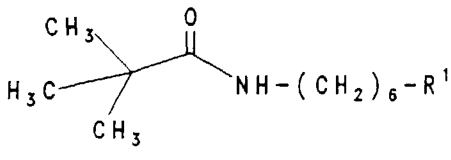

wird analog zu "Beispiel 55" aus Pivalinsäure und Amin b hergestellt.

$C_{35}H_{61}NO_6$ (591) MS. 598 (M + $Li^+$)

Beispiel 57

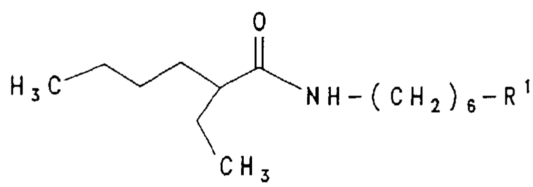

wird analog "Beispiel 55" aus 2-Ethylhexansäure und Amin b hergestellt.

$C_{38}H_{67}NO_6$ (633) MS: 640 (M + $Li^+$)

Beispiel 58

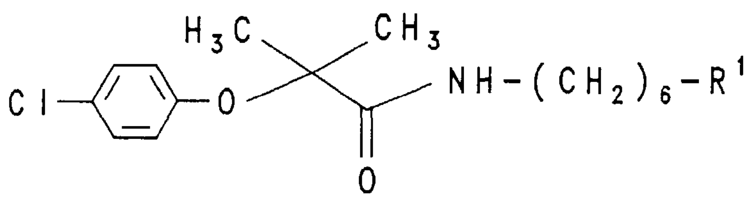

wird analog "Beispiel 55" aus Clofibrinsäure und Amin b hergestellt

$C_{40}H_{62}ClNO_7$ (703) MS: 710 (M + $Li^+$)

Beispiel 59

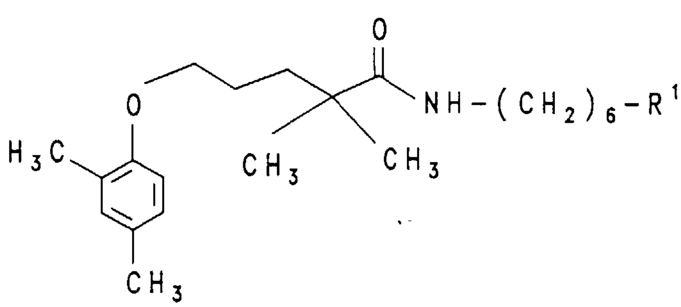

wird analog "Beispiel 55" aus Gemfibrocil und Amin b hergestellt.

$C_{45}H_{73}NO_7$ (740) MS: 747 (M + $Li^+$)

Beispiel 60

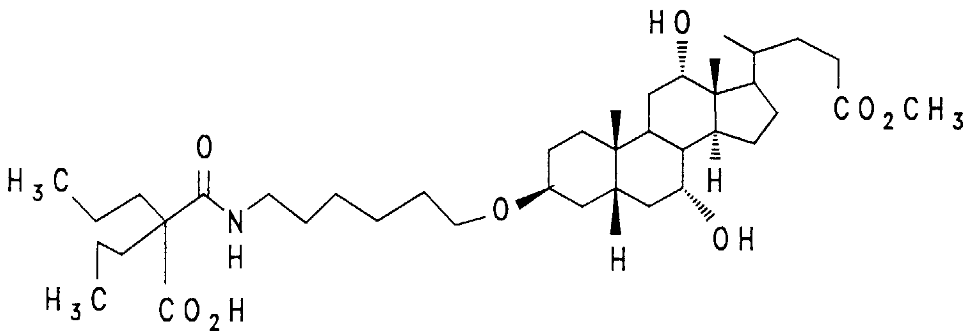

wird aus 522 mg Amin b und 94,1 mg Di-n-propylmalonsäure in THF in Gegenwart von DCC/HOBT hergestellt. Isolierung erfolgt nach 54 h. Die Ausbeute beträgt 69 %.

$C_{40}H_{69}NO_8$ (690) MS: 697 (M + $Li^+$)

Beispiel 61

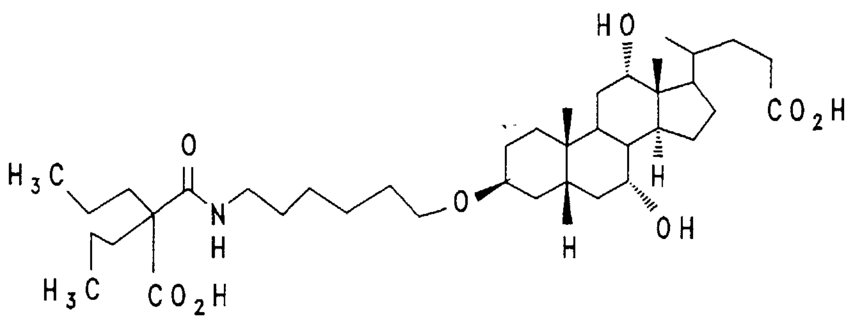

250 mg "Beispiel 60" werden in Dioxan mittels 2N NaOH verseift. Nach wäßriger Aufarbeitung und säulenchromatographischer Reinigung (EtOAc/$CH_3OH$ 10:1) erhält man 160 mg der Verbindung 61.

$C_{39}H_{67}NO_8$ (676) MS: 677 (M + 1)

## Patentansprüche

1. Monomere Gallensäurederivate der Formel I

Z-X-GS I,

in der

GS einen Gallensäurerest mit acider Funktion in der Seitenkette oder dessen Salz bedeutet,

X ein kovalente Bindung oder eine Brückgruppe der Formel $(CH_2)_n$ mit n = 1 bis 10, wobei die Alkylenkette 1 bis 3 Sauerstoffatome, NH- oder

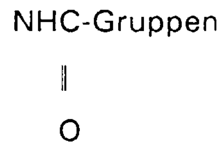

Gruppen

aufweisen kann, darstellt, wobei GS beliebig über X verbunden ist, und

Z

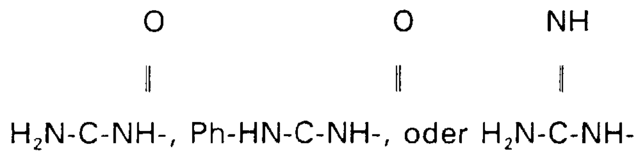

, , oder

bedeutet.

**2.** Gallensäurederivate der Formel I gemäß Anspruch 1, dadurch gekennzeichnet, daß GS in 3-Position mit X verknüpft ist, wobei die Anknüpfung in α- oder β-Stellung erfolgt.

**3.** Arzneimittel enthaltend ein Gallensäurederivat gemäß Anspruch 1.

**4.** Hypolipidaemikum enthaltend ein Gallensäurederivat gemäß Anspruch 1.

**5.** Verwendung der Verbindungen der Formel I gemäß einem der Ansprüche 1 oder 2 zur Herstellung eines Medikaments zur Behandlung von Hyperlipidämie.

## Claims

**1.** A monomeric bile acid derivative of the formula I

$$Z\text{-}X\text{-}GS \qquad I,$$

in which

GS is a bile acid radical having an acid function in the side chain or a salt thereof,
X is a covalent bond or a bridge group of the formula $(CH_2)_n$, where n = 1 to 10, in which the alkylene chain can contain 1 to 3 oxygen atoms, NH- or

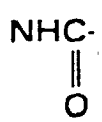

groups,

and in which GS is bonded via X as desired, and
Z is

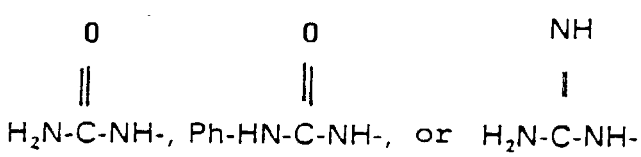

, , or

2. A bile acid derivative of the formula I as claimed in claim 1, wherein GS is linked to X in the 3-position, linking taking place in the α- or β-position.

3. A medicament comprising a bile acid derivative as claimed in claim 1.

4. A hypolipidemic agent comprising a bile acid derivative as claimed in claim 1.

5. The use of a compound of the formula I as claimed in either of claims 1 or 2 for the preparation of a medicament for the treatment of hyperlipidemia.

**Revendications**

1. Dérivés d'acides biliaires monomères de formule I

Z-X-GS    I

dans laquelle

GS représente un reste d'acide biliaire à fonction acide dans la chaîne latérale ou un sel de celui-ci,

X représente une liaison covalente ou un groupe pontant de formule $(CH_2)_n$ avec n = 1 à 10, la chaîne alkylène pouvant comporter de 1 à 3 atomes d'oxygène, groupes NH ou

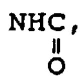

GS étant lié de façon quelconque par X, et

Z représente

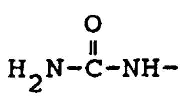 , 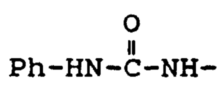 ou 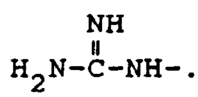

2. Dérivés d'acides biliaires de formule I selon la revendication 1, caractérisés en ce que GS est lié par X en position 3, la liaison s'effectuant en position α ou β.

3. Médicament contenant un dérivé d'acide biliaire selon la revendication 1.

4. Agent hypolipidémiant contenant un dérivé d'acide biliaire selon la revendication 1.

5. Utilisation des composés de formule I selon la revendication 1 ou 2, pour la fabrication d'un médicament destiné au traitement de l'hyperlipidémie.